# EUROPEAN PATENT SPECIFICATION

(11) **EP 2 770 324 B1**
(45) Date of publication and mention of the grant of the patent: **15.05.2019**
(21) Application number: 14156332.0
(22) Date of filing: 24.02.2014
(51) Int. Cl.: G01N 33/558, G01N 33/543

(54) **Chromatography method, chromatography kit, and method of producing an insoluble carrier for chromatography**
Chromatographieverfahren, Chromatographie-Kit und Verfahren zur Herstellung eines unlöslichen Trägers für Chromatographie
Procédé de chromatographie, kit de chromatographie et procédé de production d'un support insoluble pour la chromatographie

(30) Priority: 26.02.2013 JP 2013035334; 24.01.2014 JP 2014011088
(43) Date of publication of application: 27.08.2014
(73) Proprietor: FUJIFILM Corporation, Minato-ku Tokyo 106-8620 (JP)
(72) Inventor: Wada, Atsuhiko, Ashigarakami-gun Kanagawa 258-8577 (JP)
(74) Representative: Hoffmann Eitle

(56) References cited:
- EP-A1- 1 473 565
- EP-A2- 2 065 706
- US-A- 5 252 496
- US-A1- 2008 166 821
- US-A1- 2012 308 444
- US-B2- 7 919 653

## Description

### BACKGROUND OF THE INVENTION

### 1. Field of the Invention

The present invention relates to a chromatography method in which it is possible to detect an analysis object with high sensitivity as well as a false positive is suppressed, a chromatography kit and a method of producing an insoluble carrier for chromatography.

### 2. Description of the Related Art

Among immunoassay methods, since the operation is simple and the measurement can be performed for a short time, an immunochromatography method has been used frequently and generally. As an immune reaction used in an immunochromatography method, a competitive reaction or a sandwich type reaction has been widely used. Among those, in an immunochromatography method, a sandwich type reaction is mainly used, and in a typical example thereof, in order to detect a specimen consisting of an antigen which is an analysis object in a sample, the operation as described below is conducted. Firstly, by immobilizing the fine particles sensitized by an antibody to an antigen which is a specimen to an insoluble carrier as the solid phase fine particles or by directly immobilizing the antibody itself to an insoluble carrier, the insoluble carrier having a reactive site is prepared. On the other hand, the antibody which is able to be specifically bonded to the specimen is sensitized to the labeled fine particles to prepare the sensitized labeled fine particles. The sensitized labeled fine particles are chromatographically moved with a sample on the insoluble carrier. According to the operation described above, the antibody immobilized at the reactive site formed on the insoluble carrier becomes an immobilized reagent, and the sensitized labeled fine particles are specifically bonded thereto through the antigen which is the specimen. Then, by determining the presence or absence, or a degree of a signal generated by the sensitized labeled fine particles being captured at the reactive site by sight, the presence or absence of the existence, or the amount of the specimen in the sample can be measured.

Among the immunochromatography methods, in order to avoid a problem in which the antigen is not detected due to low sensitivity (a false negative), there are cases when a method in which a detection signal is amplified is performed. As a method of amplifying a signal, there is a method in which an enzyme such as alkaline phosphatase or peroxidase is used as a label. In addition, as a method of avoiding a problem of a false negative, a method of performing a detection by sensitizing (silver amplification) by reacting a reducing agent which can reduce a compound containing silver and an silver ion on the labeled fine particles selected from a group consisting of a metal colloid label and a metal sulfide label has been also examined. Then, in an immunographic system using a sensitized reaction, an examination in which the nonspecific adsorption is suppressed has been also conducted.

As a technique of suppressing the nonspecific adsorption, a technique in which globulin, albumin, or the like is used as a blocking agent in order to prevent a specimen which is detected in a first determination area from detecting in a second determination area in a test implement for immunochromatography having a plurality of determination areas for detecting two or more kinds of specimens, is disclosed in JP2006-189317A. In addition, a technique in which albumin, casein, polyethylene glycol or the like is used in order to suppress the nonspecific adsorption of a biologically active substance in a solid phase carrier used for a biochemical reaction chip or the like is disclosed in JP2009-229320A. Furthermore, a technique in which a fragmented antibody is used as a bonded substance on a porous carrier which is an insoluble carrier for suppressing the nonspecific adsorption in an immunochromatography method is disclosed in JP2009-216694A, in which forming a detection site by a detection substance and a nonspecific adsorption preventing agent and being able to use polyethylene glycol as an example of the nonspecific adsorption preventing agent are described.

In addition, a latex composition for immunoassay which includes at least one kind of an aggregation inhibitor or the like selected from a group consisting of the latex particles which are bonded to an antibody and/or its antigen binding fragment, saccharides and a polyalcohol is described in JP2007-315883A, in which preventing the spontaneous agglutination of the latex particles which are bonded to the antibody by using this and thus being able to perform an immunoassay having high sensitivity are described. Furthermore, an analysis method of a specimen by an immunity analysis method using a specimen, a capture antibody which is specifically bonded to the specimen, a support having a determination unit in which one kind selected from the capture antibody which is specifically bonded to the specimen is immobilized and a labeled antibody which is specifically bonded to the specimen, characterized by immobilizing a labeled substance having a sensitization action to the determination unit described above on the support described above is described in WO2007/061098, in which preventing the agglutination by forming a protective layer by polyethylene glycol, alkyl thiol, biotin, avidin, nucleic acid, or the like being adsorbed on the surface of the labeled substance is described.

On the other hand, a technique in which noise is kept low by washing a labeled substance nonspecifically absorbed at a reactive site of an insoluble carrier away using a washing liquid as a method of removing a labeled substance nonspecifically absorbed is disclosed in JP2009-139255A, JP2009-139256A, JP2009-216695A and JP2009-287952A.

### SUMMARY OF THE INVENTION

As described above, according a method of amplifying the detected signal, high sensitivity can be achieved, on the other hand, since a labeled substance nonspecifically adsorbed was also sensitized to the antibody immobilized on the insoluble carrier, noise was improved, and thus there was a problem in which a false positive risk was enhanced. To solve the problem, in a method using an immunoassay reaction or a biochemical reaction, a method of suppressing the nonspecific adsorption has been studied in the related art, however, it was still insufficient for detecting an infinitesimal specimen with high sensitivity, and in order to detect the specimen in a sample to be detected with even higher sensitivity, a development of a technique in which noise is kept even lower was desired.

The present invention provides a chromatography method in which a false positive can be specifically suppressed and a signal ratio (an S/N ratio) to very high noise can be realized, a chromatography kit and a method of producing an insoluble carrier for chromatography in a chromatography method. The present invention particularly provides a chromatography method in which a false positive due to the nonspecific adsorption is suppressed and a detection of a trace of test substance can be realized, a chromatography kit and a method of producing an insoluble carrier for chromatography in a case of using amplification technique.

As a result of intensive studies in order to solve the above problem, the present inventors found that it was possible to effectively suppress the nonspecific adsorption by immobilizing polyethylene glycol having the molecular weight from 7000 to 9000 with an antibody when an antibody was immobilized on an insoluble carrier. Furthermore, it was found that it was possible to specifically realize the suppression of a false positive by washing the nonspecific adsorption substance using a washing liquid, and in particular, the suppression of a false positive in which the suppression was particularly expected in a chromatography method using an amplification reaction was realized, and it was possible to realize a very high S/N ratio (a signal ratio to noise). The present invention has been completed based on these findings.

That is, according to the present invention, there is provided a chromatography method including a step of developing a complex of a specimen and a labeled substance modified by a first bondable substance which can be bonded to the specimen, on an insoluble carrier,
a step of capturing the complex of the specimen and the labeled substance at a reactive site on the insoluble carrier,
a step of washing the reactive site on the insoluble carrier using a washing liquid,
a step of detecting the specimen which is captured at the reactive site after a signal of the labeled substance is amplified by an amplification liquid,
in which the reactive site on the insoluble carrier includes (i) a second bondable substance which can be bonded to the specimen or a substance having affinity to the first bondable substance which can be bonded to the specimen, and (ii) polyethylene glycol having the molecular weight from 7000 to 9000, wherein the mole ratio of the polyethylene glycol and the second bondable substance which can be bonded to the specimen or the substance having affinity to the first bondable substance which can be bonded to the specimen is preferably from 12.5 to 21.3 and wherein each of the first bondable substance and the second bondable substance is an antibody.

The reactive site on the insoluble carrier is preferably provided by applying a mixed liquid including (i) the second bondable substance which can be bonded to the specimen or the substance having affinity to the first bondable substance which can be bonded to the specimen, and (ii) the polyethylene glycol onto the insoluble carrier.

The amplification liquid preferably includes a compound including silver.

The washing liquid preferably includes a reducing agent which can reduce a silver ion.

The reducing agent which can reduce a silver ion preferably includes a divalent iron ion.

The labeled substance is preferably a metal colloid.

Furthermore, according to the present invention, there is provided a chromatography kit including
(a) a labeled substance modified by a first bondable substance which can be bonded to a specimen,
(b) an insoluble carrier provided with a reactive site including (i) a second bondable substance which can be bonded to the specimen or a substance having affinity to the first bondable substance which can be bonded to the specimen and (ii) polyethylene glycol having the molecular weight from 7000 to 9000, wherein the mole ratio of the polyethylene glycol and the second bondable substance which can be bonded to the specimen or the substance having affinity to the first bondable substance which can be bonded to the specimen is preferably from 12.5 to 21.3 and wherein each of the first bondable substance and the second bondable substance is an antibody,
(c) a washing liquid, and
(d) an amplification liquid.

Furthermore, according to the present invention, there is provided a method of producing an insoluble carrier for chromatography including a step of applying a mixed liquid including (i) a second bondable substance which can be bonded to a specimen or a substance having affinity to a first bondable substance which can be bonded to the specimen and (ii) polyethylene glycol having the molecular weight from 7000 to 9000 onto an insoluble carrier, wherein the mole ratio of the polyethylene glycol and the second bondable substance which can be bonded to the specimen or the substance having affinity to the first bondable substance which can be bonded to the specimen is preferably from 12.5 to 21.3 and wherein each of the first bondable substance and the second bondable substance is an antibody.

According to the chromatography kit and the chromatography method of the present invention, by immobilizing polyethylene glycol having the molecular weight from 7000 to 9000 at the same time as well as washing the specimen using the washing liquid after the specimen is developed in the insoluble carrier and next amplifying the specimen using the amplification liquid when the antibody is immobilized to the insoluble carrier, it is possible to specifically suppress a false positive and realize a signal ratio (an S/N ratio) to very high noise.

### BRIEF DESCRIPTION OF THE DRAWINGS

Fig. 1 shows an exploded schematic view of a chromatography kit of the present invention which can be used for an assay method.
Fig. 2 shows a schematic plan view illustrating a contact state of an insoluble carrier for chromatography, an insoluble carrier for feeding a liquid and an insoluble carrier for absorbing.
Fig. 3 shows a schematic plan view illustrating a procedure of an assay method of the present invention.

### DESCRIPTION OF THE PREFERRED EMBODIMENTS

Hereinafter, description will be given of the present invention in more detail. The present invention is a chromatography method including a step of developing a complex of a specimen and a labeled substance modified by a first bondable substance which can be bonded to the specimen on an insoluble carrier, a step of capturing a complex of the specimen and the labeled substance at a reactive site on the insoluble carrier, a step of washing the reactive site on the insoluble carrier using a washing liquid, a step of detecting the specimen which is captured at the reactive site after a signal of the labeled substance is amplified by an amplification liquid, in which the reactive site on the insoluble carrier includes (i) a second bondable substance which can be bonded to the specimen or a substance having affinity to the first bondable substance which can be bonded to the specimen and (ii) polyethylene glycol having the molecular weight from 7000 to 9000. According to a method of the present invention, a preferred embodiment is to immobilize polyethylene glycol having the molecular weight from 7000 to 9000 with the antibody when a bonded substance such an antibody is immobilized to the insoluble carrier. According to this, it is possible to suppress the nonspecific adsorption. In addition, it is possible to specifically suppress a false positive which is expected to be particularly suppressed in a chromatography method using an amplification reaction by washing the nonspecific adsorption substance using the washing liquid and thus it is possible to realize a signal ratio (an S/N ratio) to very high noise.

The present invention is characterized by the reactive site on the insoluble carrier including a bonded substance such as antibody and polyethylene glycol having the molecular weight of from 7000 to 9000. In addition, a preferred embodiment is to immobilize polyethylene glycol having the molecular weight from 7000 to 9000 (in the present specification, sometimes abbreviated as PEG) with the antibody when a bonded substance such an antibody is immobilized to the insoluble carrier. If the molecular weight of polyethylene glycol is less than 7000, in a case of examining a test sample in which a test substance dose not exist, there are cases when a noise value due to the nonspecific adsorption of the labeled substance modified by the first bondable substance which can be bonded to the specimen becomes larger. Furthermore, in a case where polyethylene glycol in which the molecular weight is over 9000 is used, there are cases when a signal of the labeled substance decreases.

A measurement method of the molecular weight of polyethylene glycol is not particularly limited, and it is possible to measure the molecular weight based on a gel permeation chromatography method (a GPC method), a viscosity method, or the like. In the present specification, in a case of using commercially available polyethylene glycol, the molecular weight of polyethylene glycol is set to a value described in a catalog, and in a case of determining by actually measuring, the molecular weight of polyethylene glycol is set to a value that is measured by using a GPC (gel filtration chromatography) method and is expressed in terms of polystyrene. Gel filled in a column used in a GPC method is preferably gel having an aromatic compound as a repeating unit, and for example, gel consisting of a styrene-divinylbenzene copolymer is included. A solvent which is used in the GPC method includes an ether-base solvent such as tetrahydrofuran, a amide-based solvent such as N-methylpyrrolidone, a halogen-based solvent such as chloroform, and an aromatic solvent such as 1,2-dichlorobenzene.

As polyethylene glycol used in the present invention, commercial products can be used, and for example, the following ones can be used. (model number P5413) manufactured by Sigma-Aldrich Co. LLC and (model number 0159) manufactured by AMRESCO Inc. as polyethylene glycol having the molecular weight of 8,000is included. These polyethylene glycols may be used alone or as a combination of two or more kinds thereof.

The mole ratio of polyethylene glycol having the molecular weight from 7000 to 9000 and the second bondable substance which can be bonded to the specimen or the substance having affinity to the first bondable substance which can be bonded to the specimen (PEG/the second bondable substance which can be bonded to the specimen or the substance having affinity to the first bondable substance which can be bonded to the specimen) is from 12.5 to 21.3.

The amount of polyethylene glycol included in the reactive site on the insoluble carrier is not particularly limited, however, is preferably 0.0072 µg/mm² to 1.7 µg/mm², is further preferably 0.072 µg/mm² to 0.57 µg/mm², and is most preferably 0.10 µg/mm² to 0.50 µg/mm².

A method of including polyethylene glycol in the reactive site on the insoluble carrier is not particularly limited as long as an effect of the present invention can be obtained, however, it is preferred that the reactive site on the insoluble carrier be provided by applying a mixed liquid including polyethylene glycol and the second bondable substance which can be bonded to the specimen or the substance having affinity to the first bondable substance which can be bonded to the specimen onto the insoluble carrier. Moreover, an embodiment in which polyethylene glycol is applied after the second bondable substance which can be bonded to the specimen or the substance having affinity to the first bondable substance which can be bonded to the specimen is immobilized is not preferable, from the viewpoint of achieving an effect of the present invention, and an embodiment in which the second bondable substance which can be bonded to the specimen or the substance having affinity to the first bondable substance which can be bonded to the specimen and polyethylene glycol are applied at the same time is preferable in the present invention.

### 1. Chromatography

The present invention is to achieve the problem described above in a chromatography method of amplifying a detection signal. A chromatograph method is generally a technique of simply and quickly, or specifically determining or measuring a specimen by a technique as described below. That is, a chromatography carrier (a part of the insoluble carrier) provided with the labeled substance capture area including at least one reactive site having a bonded substance (equivalent to the second bondable substance which can be bonded to the specimen or the substance having affinity to the first bondable substance described below. specifically, an antibody, an antigen or the like.) which is capable of being bonded to the specimen is used as a solid phase. A liquid including the labeled substance modified by the first bondable substance which can be bonded to the specimen is chromatographically moved as a moving phase on the chromatograph carrier. According to this, while the specimen and the labeled substance is specifically being bonded, the specimen and the labeled substance reach the labeled substance capture area having the reactive site. At the reactive site of the labeled substance capture area, by a complex of the specimen and the labeled substance being specifically bonded to an immobilized bonded substance (the second bondable substance or the substance having affinity to the first bondable substance), only in a case where the specimen exists in a test sample, the labeled substance is concentrated in the bonded substance (the second bondable substance or the substance having affinity to the first bondable substance). As to the labeled substance, by measuring the amount of label substance visually or by using an appropriate apparatus, the presence of the specimen in the test sample is qualitatively and quantitatively analyzed.

In a chromatography method in the present invention, the reactive site was washed by using the washing liquid after a complex of the specimen and the labeled substance is specifically bonded to the immobilized bonded substance (the second bondable substance or the substance having affinity to the first bondable substance) at the reactive site of the labeled substance capture area, afterward, a signal is amplified by using the amplification liquid. An amplification reagent which is used for amplifying the signal of the labeled substance (preferably, two kinds of amplification reagents, for example, preferably, a reducing agent which can reduce a silver ion and a compound containing silver) is used, the complex of the specimen and the labeled substance which is bonded to the immobilization agent on the labeled substance capture area is set to as a core, a signal is amplified by an amplification reaction, and as a result, it is possible to achieve high sensitivity. According to the present invention, it is possible to perform chromatography with quick and high sensitivity.

### 2. Test sample

The test sample which can be analyzed by using the chromatography method and the kit of the present invention is not particularly limited as long as the test sample is a sample in which there is a possibility to contain the specimen, for example, a biological sample, in particular, body fluids (for example, blood, serum, plasma, cerebrospinal fluid, tears, sweat, urine, pus, nasal mucus, or sputum), excrement (for example, feces), organs, tissues, mucous membrane, skin, scraping test substances (swabs) which are considered that they are contained, mouth washings of animals (particularly human beings), or animals and plants themselves or dried bodies thereof can be included. As the specimen, for example, biologically active substances such as natural products, toxins, hormones or pesticides, environmental pollutants, viruses, antigens, antibodies and the like are include.

### 3. Pretreatment of test sample

In the chromatography method of the present invention, the test sample can be used as it is, as a form of an extraction liquid obtained by extracting the test sample using an appropriate solvent for extraction, as a form of a diluted liquid obtained by diluting an extraction liquid with an appropriate diluent, or as a form in which an extraction liquid is concentrated by an appropriate method. As a solvent for extraction used in the present invention, a solvent which is usually used in an immunological analysis method (for example, water, an isotonic sodium chloride solution, a buffer solution, or the like) or a water miscible organic solvent which can directly conduct an antigen-antibody reaction by diluting with the solvent can be also used.

### 4. Configuration

In the chromatography kit for performing the chromatography method of the present invention, it is possible to use by incorporating a strip for chromatograph. As a strip for chromatography which can be used, there is no particular limitation, as long as the strip for chromatography is a strip for chromatography which can be used in a usual chromatography method.

The strip for chromatography which can be used in the present invention has a labeled substance retention area and a labeled substance capture area from the upper stream direction toward the lower stream direction of the developing direction of the test sample containing the specimen. For example, an embodiment in which a sample addition pad, a labeled substance retention pad having the labeled substance retention area (for example, gold colloid antibody retention pad), a bonded substance immobilized membrane which is the insoluble carrier (for example, a chromatography carrier having the labeled substance capture area) and a absorbent pad are arranged on an adhesive sheet in this order is preferably used. The chromatography carrier (the insoluble carrier) on which the bonded substance is immobilized has the labeled substance capture area which is an area having at least one reactive site (test line) at which an antibody or an antigen which is specifically bonded to the specimen is immobilized, and may further have a control area which is an area having at least one control line in which an antibody or an antigen for controlling is immobilized, as desired.

The labeled substance retention pad having the labeled substance retention area which can be used in the present invention can be prepared by preparing a suspension containing the labeled substance and applying the suspension onto an appropriate absorbent pad (for example, glass fibre pad), afterward, drying it.

### 4-1. Labeled substance

As the labeled substance (the label which is used for labeling the first bondable substance which is specifically bonded to the specimen (the antigen)) used in the present invention, the labeled substance containing a metal is preferably used. As the kinds of metals which can be used in the present invention, a noble metal such as gold, silver or platinum, iron, lead, copper, cadmium, bismuth, antimony, tin or mercury can be preferably used, and a noble metal such as gold, silver or platinum can be further preferably used. As the preferred form of the labeled substance containing a metal can be used in the present invention, a metal colloid label or a metal sulfide label can be used. As a metal colloid label, a platinum colloid, a gold colloid, a silver colloid, an iron colloid, an aluminum hydroxide colloid, or the like can be preferably used, and as a metal sulfide label, each sulfide such as iron, silver, lead, copper, cadmium, bismuth, antimony, tin or mercury can be preferably used. In the present invention, a platinum colloid, a gold colloid, or a silver colloid can be further preferably used, and a gold colloid can be most preferably used. In a case where the gold colloid particles are used as the metal colloid label, a commercially available one may be used. Alternatively, the gold colloid particles can be prepared by using a usual method, for example, a method of reducing chloroauric acid by sodium citrate (Nature Physical Science, 241 (1973) 20, or the like).

The average particle diameter of a metal colloid is preferably from approximately 1 nm to 500 nm, further preferably from 3 nm to 100 nm, and particularly preferably from 5 nm to 60 nm. The average particle diameter of a metal colloid which is used in the present invention can be measured by a commercially available particle size distribution analyzer or the like. As a measurement method of the particle size distribution, an optical microscopy, a confocal laser microscopy, an electron microscopy, an atomic force microscopy, a static light scattering method, a laser diffraction method, a dynamic light scattering method, a centrifugal sedimentation method, an electrical pulse measurement method, a chromatography method, an ultrasonic attenuation method, and the like are known and apparatuses corresponding to each principle are commercially available.

Due to diameter range and ease of measuring, a dynamic light scattering method can be preferably used in the present invention. As a commercially available measuring apparatus using dynamic light scattering, Nanotrac UPA (manufactured by NIKKISO CO., LTD.), a dynamic light scattering particle size distribution measuring apparatus LB-550 (manufactured by HORIBA, Ltd.), Fiber-optics particle analyzer FPAR-1000 (manufactured by OTSUKA ELECTRONICS CO., LTD), and the like are included, and in the present invention, the average particle diameter is determined as a value of median size (d=50) measured at measurement temperature of 25°C.

In chromatography using a metal colloid label, a metal sulfide label, other metal alloy label (hereinafter, sometimes referred to as a metal-based label), or a polymer particle label containing a metal as the labeled substance for detecting, it is possible to amplify a signal of a metal-based label. Specifically, after the complex of the specimen and the labeled substance is formed, when a silver ion which is supplied from a compound which contains silver such as an inorganic silver salt or an organic silver salt comes into contact with the reducing agent which can reduce a silver ion and the silver particles are generated by a silver ion being reduced by the reducing agent, the metal-based label is set to a core and the silver particles are deposited on the metal-based label, therefore, the metal-based label is amplified, and thus it is possible to conduct an analysis of the specimen with high sensitivity. Therefore, in the chromatography method of the present invention, it is possible to apply the conventionally known chromatography method as it is in other respects, except conducting a reaction of depositing onto a label of an immune complex and analyzing a signal amplified in this manner by using the silver particles generated by the reduction action of a silver ion by the reducing agent.

### 4-2. Bonded substance

In the present invention, the labeled substance is modified by the first bondable substance which can be bonded to the specimen. The first bondable substance is an antibody to a specimen (an antigen).

The first bondable substance is an antibody and the second bondable substance is an antibody. The first bondable substance is more preferably an antibody and the second bondable substance is an antibody which is bonded to the first bondable substance.

The antibody having specificity to the specimen which is used in the chromatography method of the present invention is not particularly limited, however, for example, an antiserum prepared from a serum of an animal immunized by the specimen, a immunoglobulin fraction refined from an antiserum, a monoclonal antibody obtained by cell fusion using a spleen cell of an animal immunized by the specimen, or a fragment thereof (for example, F(ab')₂, Fab, Fab', or Fv) can be used. The preparation of these antibodies can be conducted by a usual method.

In the present invention, among the methods of modifying the labeled substance by using the first bondable substance, for example, as a method in which the metal colloid is bonded to the specific bonded substance, a conventionally known method (for example, The Journal of Histochemistry and Cytochemistry, 30,7 (1982) 691-696) described below can be included. As a specific example, the metal colloid and the specific bonded substance (for example, the antibody) are mixed in an appropriate buffer solution for 5 minutes or longer at room temperature. After the reaction, by dispersing a precipitation obtained by centrifugation in a solution containing a dispersing agent such as polyethylene glycol, an objective substance can be obtained.

### 4-3. Insoluble carrier

As the insoluble carrier which can be used in the present invention, a porous carrier is preferable. A nitrocellulose membrane, a cellulose membrane, a cellulose acetate membrane, a polysulfone membrane, a polyethersulfone membrane, a nylon membrane, a glass fiber, a non-woven fabric, a cloth, a thread and the like are particularly preferable.

In the present invention, the reactive site in which the second bondable substance which can be bonded to the specimen or the substance having affinity to the first bondable substance which can be bonded to the specimen and polyethylene glycol having the molecular weight from 1,500 to 10,000 are immobilized is included in the labeled substance capture area of the insoluble carrier for chromatography. The reactive site may be formed by directly immobilizing the second bondable substance which can be bonded to the specimen or the substance having affinity to the first bondable substance to a part of the insoluble carrier by a physical or chemical bond, or the reactive site may be also formed by immobilizing by being physically or chemically bonded to fine particles such as latex particles and trapping the fine particles to a part of the insoluble carrier. Moreover, after the second bondable substance or the substance having affinity to the first bondable substance is immobilized, it is preferred that the insoluble carrier be subjected to a nonspecific adsorption prevention treatment by a treatment using inactive protein or the like to be used. An embodiment in which the insoluble carrier of the present invention has plural kinds of the reactive site can also be preferably used, furthermore, the control site described above may be also include as a part of the labeled substance capture area, as desired.

### 4-4. Labeled substance retention pad

The insoluble carrier (the insoluble carrier for chromatography) of the present invention is preferably used by incorporating a labeled substance retention pad having the labeled substance retention area. As a labeled substance retention pad, a gold colloid retention pad can be included. As a material of the labeled substance retention pad, for example, a cellulose filter paper, a glass fiber, a non-woven fabric, and the like can be preferably used. The labeled substance retention pad can be manufactured by impregnating the material described above with the labeled substance prepared as described above with a certain amount and next drying the material.

### 4-5. Sample addition pad

The insoluble carrier (the insoluble carrier for chromatography) of the present invention is preferably used by further incorporating a sample addition pad. An embodiment in which the sample addition pad has functions of not only accepting the sample (the test sample) containing the added specimen but also filtrating the insoluble matter particles or the like in the sample is preferable. As the quality of the material of the sample addition pad, ones having uniform characteristics such as a cellulose filter paper, a glass fiber, polyurethane, polyacetate, cellulose acetate, nylon, cotton are included. In addition, when analyzing, in order to prevent the accuracy of analysis from reducing by the specimen in the sample nonspecifically being adsorbed to the quality of the material of the sample addition pad, the quality of the material configuring the sample addition unit can be also used by being subjected to a nonspecific adsorption prevention treatment in advance. In the present invention, the sample addition pad may work as the labeled substance retention pad having the labeled substance retention area described in 4-4.

### 4-6. Absorbent pad

In the present invention, it is possible to use the absorbent pad by preferably incorporating in the insoluble carrier (a strip for chromatography). The absorbent pad is a site in which the added test sample which chromatographically moves is physically absorbed as well as the labeled substance or the like which is not captured at the reactive site of the chromatography carrier is absorbed and removed, and a water absorbency material such as a cellulose filter paper, a non-woven fabric, a cloth, cellulose acetate is used. After the added test sample chromatographically moves and reaches the absorbent pad, since the speed of chromatographically moving differs depending on the quality of the material, size or the like of the absorbent pad, it is possible to set the speed which is suited to the measurement of the specimen by the selection.

### 5. Washing liquid

In the present invention, the washing liquid is not particularly limited as long as the washing liquid is a liquid which has a function of washing the labeled substance which remains (that is, nonspecifically remains) in the insoluble carrier except a specific bonding reaction, and in order to enhance a washing effect, pH may be adjusted or a washing liquid in which a high molecular compound such as a surfactant component or protein such as BSA is added may be used. In the present invention, a liquid containing a reducing agent which can reduce a silver ion explained below is preferably used as a washing liquid. In this case, one of the amplification liquids out of two kinds of the amplification liquid used (described later) also plays a role as a washing liquid.

Since the washing liquid is developed while washing the labeled substance which nonspecifically remains during developing, the washing liquid is developed while containing the labeling substance, however, as the washing liquid before developing, a liquid which does not contain the labeled substance is preferably used in order to enhance a washing effect.

After the test sample (the test substance liquid) is developed, the washing liquid is added to the insoluble carrier, and a labeled matter which remains in the insoluble carrier, except a labeled matter bonded by a specific bonding reaction is washed. As a method of feding a liquid of the washing liquid, a method in which the washing liquid is added to a sample dripping unit as it is after the test substance liquid is developed, a method in which a insoluble carrier for feeding a liquid (a washing liquid addition pad) for feeding a liquid of the washing liquid and an insoluble carrier for absorbing (a absorption pad) are adhered to the insoluble carrier in advance, the washing liquid is added to the insoluble carrier for feeding a liquid and a liquid is fed in the insoluble carrier for absorbing direction, and a method in which the addition site of the washing liquid is provided in the insoluble carrier in advance and the washing liquid is added to the addition site of the washing liquid after a test substance liquid is developed are included, or the insoluble carrier for feeding a liquid for feeding a liquid of the washing liquid and the insoluble carrier for absorbing may be adhered to the insoluble carrier after the test substance liquid is developed in the insoluble carrier.

In the present invention, an angle formed by the developing direction of the test sample (the test substance liquid) and the developing direction of the washing liquid is not particularly limited, and it is possible to performed from at 0 degrees to 180 degrees, however, it is preferred to perform at 90 degrees. The insoluble carrier for feeding a liquid is not particularly limited as long as it is possible to add the washing liquid, and a glass fiber pad, a cellulose membrane, a nitrocellulose membrane, and the like can be used.

The insoluble carrier for absorbing is not particularly limited as long as the insoluble carrier for absorbing is a substance capable of absorbing, and cellulose, nitrocellulose, a glass fiber, a mixture thereof, and the like can be used.

### 6. Method of immunological examination

Hereinafter, as to the chromatography method of the present invention, description will be given of a sandwich method which is a specific embodiment thereof.

The sandwich method is not particularly limited, however, for example, an analysis of the specimen can be conducted by the following procedure. Firstly, the first bondable substance (for example, the first antibody) and the second bondable substance (for example, the second antibody) having specificity to the specimen (antigen) are prepared in advance by the method described before. In addition, the labeled substance is modified by the first bondable substance in advance. When the second bondable substance is immobilized on an appropriate carrier for chromatography (an insoluble carrier) (for example, a nitrocellulose membrane, a glass fiber membrane, a nylon membrane, a cellulose membrane, or the like) to be set to labeled substance capture area and comes into contact with the test sample (or the extraction thereof) in which there is a possibility to contain the specimen (antigen), in a case where the specimen exists in the test sample, the bonding with the second bondable substance (for example, the antigen-antibody reaction with the second antibody) occurs. At the same time as or after the bonding of the specimen and the second bondable substance, by further coming into contact with the labeled substance modified by the first bondable substance with an excess amount, in a case where the specimen exists in the test sample, a complex consisting of the immobilized second bondable substance, the specimen (antigen) and the labeled substance modified by the first bondable substance is formed.

In the sandwich method, after finishing the reaction of the second bondable substance and the specimen (antigen) which are immobilized and the first bondable substance which modifies the specimen and the labeled substance, the labeled substance in which an immune complex is not formed is removed, subsequently, for example, by observing the labeled substance capture area of the insoluble carrier as it is and detecting or determining the quantity of the labeled substance, it is possible to determine the presence or absence of, or measure the quantity of the specimen in the test sample. In the present invention, for example, by supplying the reducing agent and the compound containing a silver ion, a signal from the labeled substance which forms the complex is amplified and detected.

### 7. Amplification liquid

The amplification liquid used in the present invention is a liquid containing an amplification reagent. The amplification reagent is a reagent in which the colored compound, the emission or the like is generated and the amplification of a signal can occur by catalytically reacting by the action of the labeled substance and the specimen, and can be used in a state of the solution containing the reagent, that is, as the amplification liquid. For example, a silver ion solution in which the eduction of metal silver occurs on the metal label by a physical development, a solution of a phenylenediamine compound and a naphthol compound which becomes a pigment by the action of a peroxidase label and hydrogen peroxide, and the like are included.

In detail, it is possible to use the so-called developer as described in general books in a field of photographical chemistry (for example, "Revised Fundamentals of Photographic engineering-silver-halide photo edition-" (edited by The Society of Photography and Imaging of Japan, CORONA PUBLISHING CO., LTD.), "Chemistry of photograph" (SASAI akira, Shyshinkogyo publisher), and "Latest Photographic prescription Handbook" (KIKUCHI shinich and others, Amiko publisher)) as an amplification liquid containing an amplification reagent. That is, it is possible to use as an amplification liquid without particular limitation as long as the amplification liquid is the so-called physical developer in which a metal colloid and the like as a silver ion is contained in a liquid and a silver ion in a liquid becomes a core of a development are mainly reduced.

In the present invention, two kinds of amplification reagents can be used. Among two kinds of amplification reagents used for amplifying a signal of the labeled substance captured in the labeled substance capture area, it is preferred that a first amplification liquid contain a first amplification reagent and a second amplification liquid contain a second amplification reagent, and the amplification be performed by serially adding the first amplification liquid and the second amplification liquid.

As a specific example of the amplification liquid, a combination of the first amplification liquid containing the reducing agent which can reduce a silver ion and the second amplification liquid containing the compound which contains silver can be used.

Hereinafter, description will be given of the reducing agent which can reduce a silver ion contained in the first amplification liquid, the compound containing silver contained in the second amplification liquid, and the like.

### 7-1. Compound containing silver

As a compound containing silver, a silver ion containing compound, for example, an organic silver salt, an inorganic silver salt, or a silver complex can be used. A silver ion containing compound having high solubility with respect to a solvent such as water is preferable, and silver nitrate, silver acetate, silver lactate, silver butyrate, silver thiosulfate, and the like are included. Silver nitrate is particularly preferable. As a silver complex, a silver complex which is coordinated to a ligand having a water-soluble group such as a hydroxyl group or a sulfone group is preferable, and hydroxy thioether silver and the like are included.

The inorganic silver salt or the silver complex from 0.001 mol/m² to 0.2 mol/m² is generally contained and from 0.01 mol/m² to 0.05 mol/m² is preferably contained, as silver.

### 7-2. Reducing agent which can reduce silver ion

As long as the reducing agent which can reduce a silver ion can reduce a silver ion to silver, any inorganic or organic material, or even a mixture thereof can be used.

As an inorganic reducing agent, a reducing metal salt and a reducing metal complex salt in which a valence can be changed by a metal ion such as Fe²⁺, V²⁺ or Ti³⁺ are preferably included. When the inorganic reducing agent is used, it is necessary to remove or detoxify by performing a complexation of, or reducing an ion which is oxidized. For example, in a system in which Fe²⁺ is used as a reducing agent, it is possible to detoxify by a complex of Fe³⁺ which is an oxide being formed by using citric acid or EDTA. In the present system, such the inorganic reducing agent is preferably used, and a metal salt of Fe²⁺ is more preferable.

Moreover, a developing agent used for a wet silver halide photographic sensitive material (for example, a methyl gallate, hydroquinone, substituted hydroquinone, 3-pyrazolidones, p-aminophenols, p-phenylenediamines, hindered phenols, amidoximes, azines, catechols, pyrogallols, ascorbic acid (or a derivative thereof) and leuco dyes), and other materials which are evident to persons skilled in the art, for example, a material described in US6020117A can be also used.

As the reducing agent, an ascorbic acid reducing agent is also preferable. A useful ascorbic acid reducing agent contains an analogue and an isomer of ascorbic acid, and a derivative thereof, for example, D- or L-ascorbic acid and a sugar derivative thereof (for example, γ-lactoascorbic acid, glucoascorbic acid, fucoascorbic acid, glucoheptoascorbic acid, martascorbic acid), a sodium salt of ascorbic acid, a potassium salt of ascorbic acid, isoascorbic acid (or L-erythroascorbic acid) and a salt thereof (for example, an alkali metal salt, an ammonium salt, or a salt which is known in the art), an endiol-type ascorbic acid, an enaminol-type ascorbic acid, a thioenol-type ascorbic acid, and the like are preferably included, D- or L-ascorbic acid (and an alkali metal salt thereof) or isoascorbic acid (or an alkali metal salt thereof) is particularly preferable, and a sodium salt is a preferred salt. A mixture of these reducing agents can be used, as necessary.

### 8. Other auxiliary agent

As other auxiliary agent of the amplification liquid, there are cases when a buffering agent, a preservative (for example, an antioxidant or an organic stabilizer) and a speed regulating agent are contained. As a buffering agent, for example, a buffering agent using acetic acid, citric acid, sodium hydroxide or a salt of any of these, or tris(hydroxymethyl)aminomethane, and in addition, a buffering agent which is used in a general chemical experiment can be used. It is possible to adjust to an optimum pH for the amplification liquid by appropriately using these buffering agents. In addition, alkylamine as an antifogging agent can be used as an additive agent, and dodecylamine is particularly preferable. In addition, in order to improve the solubility of these additive agents, a surfactant can be used, and C₉H₁₉-C₆H₄-O-(CH₂CH₂O)₅₀H is particularly preferable.

As a method of spotting the amplification reagent onto chromatography kit, a method in which the reducing agent solution as the first amplification liquid is spotted on a pad for feeding a liquid of the reducing agent solution, a silver ion solution area as the second amplification liquid is spotted on an area containing the labeled substance capture from the above, and the silver ion solution is infiltrated in the thickness direction of the insoluble carrier is preferable.

As a method of embedding two kinds of amplification reagents in the chromatography kit, a method of arranging a pot which contains the solution containing each amplification reagent on top of site at which each amplification reagent is spotted is include. It is preferred that the reducing agent solution (the first amplification liquid) be placed on the top of the pad for feeding a liquid of the reducing agent solution and the pot containing the silver ion solution (the second amplification liquid) be arranged immediately above the silver ion solution packing hole. By arranging in this manner, a liquid flows by pushing each pot and thus it is possible to spot at a predetermined site.

### 9. Chromatography kit

The chromatography method of the present invention can be conducted by using the chromatography kit provided with the labeled substance modified by the first bondable substance which can be bonded to the specimen and the insoluble carrier containing the second bondable substance which can be bonded to the specimen or the substance having affinity to the first bondable substance which can be bonded to the specimen. In this case, in the chromatography kit, the labeled substance modified by the first bondable substance which can be bonded to the specimen may be provided on the insoluble carrier in advance. Alternatively, the labeled substance modified by the first bondable substance which can be bonded to the specimen may be provided apart from the insoluble carrier. In this case, the measurement can be performed by a method of developing on the insoluble carrier after the labeled substance which is provided apart from the insoluble carrier is mixed with the test sample, or the like. The chromatography kit of the present invention includes a washing liquid and an amplification liquid, and preferably, an amplification liquid which contains a compound containing silver and a reducing agent which can reduce a silver ion can be provided. As to the examples and the preferred ranges of each material configuring the chromatography kit, the examples and the ranges described in the chromatography method and the like can be preferably used.

In particular, according to the present invention, there is provided a chromatography kit including
(a) a labeled substance modified by the first bondable substance which can be bonded to the specimen,
(b) an insoluble carrier provided with a reactive site containing (i) a second bondable substance which can be bonded to the specimen or a substance having affinity to the first bondable substance which can be bonded to the specimen and (ii) polyethylene glycol having the molecular weight from 1,500 to 10,000,
(c) a washing liquid, and
(d) an amplification liquid.

The labeled substance containing a metal modified by the first bondable substance which can be bonded to the specimen may be arranged on the labeled substance retention pad or may be provided apart from the labeled substance retention pad.

Furthermore, according to the present invention, there is provided a method of producing an insoluble carrier for chromatography including applying a mixed liquid containing (i) the second bondable substance which can be bonded to the specimen or the substance having affinity to the first bondable substance which can be bonded to the specimen and (ii) polyethylene glycol having the molecular weight from 1,500 to 10,000 onto the insoluble carrier. The insoluble carrier for chromatograph produced by the method describe above can be used in the chromatography method and the chromatography kit according to the present invention.

Description will be given of the present invention in more detail according to the following Examples, however, the present invention is not limited to Examples.

### [Example]

### (1) Manufacturing of influenza virus antigen detection immunochromatography kit

### (1-1) Manufacturing of anti-influenza A type antibody modifying gold colloid

### (1-1-1) Manufacturing of F(ab')₂ fragmented anti-influenza A type virus antibody

160 µg/mL of F(ab')₂ fragmented anti-influenza A type virus antibody solution was manufactured from Anti-influenza A type virus antibody (the stock number 7307, manufactured by Medix Biochemia) by using ImmunoPureIgG1 Fab and F(ab')₂ Preparation Kit (the stock number 44880, manufactured by Pierce Biotechnology, Inc.).

### (1-1-2) Manufacturing of anti-influenza A type fragmented antibody modifying gold colloid

1 mL of the F(ab')₂ fragmented anti-influenza A type virus antibody solution manufactured in (1-1-1) was added to a gold colloid solution in which pH was adjusted by adding 1 mL of 50 mM KH₂PO₄ buffer (pH 7.5) to 9 mL of a gold colloid solution having a diameter of 50 nm (EM.GC50, manufactured by BB International Ltd.) and stirred. After leaving to stand for 10 minutes, 550 µL of 1% by mass of polyethylene glycol (PEG the molecular weight 20,000, the stock number 168-11285, manufactured by Wako Pure Chemical Industries, Ltd.) aqueous solution was added thereto and stirred, subsequently, 1.1 mL of 10% by mass of bovine serum albumin (BSA Fraction V, the stock number A-7906, manufactured by Sigma-Aldrich Co. LLC) aqueous solution was added thereto and stirred. After the solution was centrifuged for 30 minutes under 8,000×g at 4°C (himacCF16RX, manufactured by Hitachi Koki Co., Ltd.), a supernatant was removed while approximately 1 mL remained, and the remaining solution was sonicated with an ultrasonic cleaning device to redisperse the gold colloid. After the solution was dispersed in 20 mL of a gold colloid preservation solution (20 mM of Tris-HCl buffer (pH 8.2), 0.05% by mass of PEG (the molecular weight 20,000), 150 mM of NaCl and 1% by mass of BSA) and the obtained mixture was centrifuged for 30 minutes under 8,000×g at 4°C again, a supernatant was removed while approximately 1 mL remained, the remaining solution was sonicated with an ultrasonic cleaning device to redisperse the gold colloid , and an antibody modifying gold colloid (50 nm) solution was obtained.

### (1-2) Manufacturing of anti-influenza B type antibody modifying gold colloid

### (1-2-1) Manufacturing of F(ab')₂ fragmented anti-influenza B type virus antibody

60 µg/mL of F(ab')₂ fragmented anti-influenza B type virus antibody solution was manufactured from Anti-influenza B type virus antibody (the stock number M2110171, manufactured by Fitzgerald Industries International, Inc.) by using V8-protease (the stock number 164-13982, manufactured by Wako Pure Chemical Industries, Ltd.).

### (1-2-2) Manufacturing of anti-influenza B type fragmented antibody modifying gold colloid

1 mL of the F(ab')₂ fragmented anti-influenza B type antibody solution manufactured in (1-2-1) was added to a gold colloid solution in which pH was adjusted by adding 1 mL of 20 mM Borax buffer (pH 9.0) to 9 mL of a gold colloid solution having a diameter of 50 nm (EM.GC50, manufactured by BBinternational Ltd.) and stirred. After leaving to stand for 10 minutes, 550 µL of 1% by mass of polyethylene glycol (PEG the molecular weight 20,000, the stock number 168-11285, manufactured by Wako Pure Chemical Industries, Ltd.) aqueous solution was added thereto and stirred, subsequently, 1.1 mL of 10% by mass of bovine serum albumin (BSA Fraction V, the stock number A-7906, manufactured by Sigma-Aldrich Co. LLC) aqueous solution was added thereto and stirred. After the solution was centrifuged for 30 minutes under 8,000×g at 4°C (himacCF16RX, manufactured by Hitachi Koki Co., Ltd.), a supernatant was removed while approximately 1 mL remained, and the remaining solution was sonicated with an ultrasonic cleaning device to redisperse the gold colloid. After the solution was dispersed in 20 mL of a gold colloid preservation solution (20 mM of Tris-HCl buffer (pH 8.2), 0.05% by mass of PEG (the molecular weight 20,000), 150 mM of NaCl and 1% by mass of BSA) and the obtained mixture was centrifuged for 30 minutes under 8,000×g at 4°C again, a supernatant was removed while approximately 1 mL remained, the remaining solution was sonicated with an ultrasonic cleaning device to redisperse the gold colloid, and an antibody modifying gold colloid (50 nm) solution was obtained.

### (1-3) Manufacturing of anti-influenza A type antibody modifying gold colloid retention pad

The anti-influenza A type fragmented antibody modifying gold colloid manufactured in (1-1-2) was diluted by using a gold colloid application liquid (20 mM of Tris-HCl buffer (pH 8.2), 0.05% by mass of PEG (the molecular weight 20,000) and 5% by mass of sucrose) and water so that absorbance at 520 nm became 1.5. The solution was uniformly applied with 1.1 mL each per one sheet of a glass fiber pad (Glass Fiber Conjugate Pad, manufactured by EMD Millipore Corporation) which was cut by 8 mm×200 mm, and drying under reduced pressure was performed for 12 hours to obtain a gold colloid antibody retention pad. The part of retaining the gold colloid antibody is equivalent to the labeled substance retention area.

### (1-4) Manufacturing of anti-influenza B type antibody modifying gold colloid retention pad

The anti-influenza B type fragmented antibody modifying gold colloid manufactured in (1-2-2) was diluted by using a gold colloid application liquid (20 mM Tris-HCl buffer (pH 8.2), 0.05% by mass of PEG (the molecular weight 20,000) and 5% by mass of sucrose) and water so that absorbance at 520 nm became 1.5. The solution was uniformly applied with 1.1 mL each per one sheet of a glass fiber pad (Glass Fiber Conjugate Pad, manufactured by EMD Millipore Corporation) which was cut by 8 mm×200 mm, and drying under reduced pressure was performed for 12 hours to obtain a gold colloid antibody retention pad. The part of retaining the gold colloid antibody is equivalent to the labeled substance retention area.

### (1-5) Manufacturing of anti-influenza A type antibody immobilized membrane (chromatography carrier)

### (1-5-1) Example 1 (Reference)

By using a nitrocellulose membrane which was cut by 25 mm×200 mm (there is a plastic-backed, HiFlow Plus HF120, manufactured by EMD Millipore Corporation), an antibody was immobilized by a method as described below to manufacture an antibody immobilized membrane. The long side of the membrane was set to the lower side (the near side), and at a position of 7 mm from the lower side, a solution in which polyethylene glycol having the molecular weight of 2,000 was mixed with anti-influenza A type monoclonal antibody for immobilization (Anti-Influenza A SPTN-5 7307, manufactured by Medix Biochemica) solution which was prepared so as to be 1.5 mg/mL so that the content of polyethylene glycol became 0.1% by mass was applied in a line shape having the width of approximately 0.7 mm by using an ink jet type applying device (manufactured by BioDot, Inc.). This line is called a test line. In the same way, at a position of 13 mm from the lower side, an anti-mouse IgG antibody for control (an anti-mouse IgG (H+L), rabbit F(ab')₂, the stock number 566-70621, manufactured by Wako Pure Chemical Industries, Ltd.) solution which was prepared so as to be 0.5 mg/mL was applied in a line shape. This line is called a control line. The applied membrane was dried at 50°C for 30 minutes by a hot air type dryer. 500 mL of a blocking liquid (50 mM boric acid buffer (pH 8.5) containing 0.5% by mass of casein (derived from the milk, the stock number 030-01505, manufactured by Wako Pure Chemical Industries, Ltd.)) was put into the vat, and the dried membrane was immersed therein and was left to stand for 30 minutes as it was. Afterward, the membrane which was taken out from a blocking liquid was immersed in 500 mL of a washing and stabilized liquid (50 mM Tris-HCl (pH 7.5) buffer containing 0.5% by mass of sucrose and 0.05% by mass of sodium cholate) which was put into another vat and was left to stand for 30 minutes as it was. The membrane was taken out from the liquid and dried at room temperature for 12 hours to set to an antibody immobilized membrane.

### (1-5-2) Example 2 (Reference)

The antibody immobilized membrane was manufactured in the same way as (1-5-1) except using polyethylene glycol having the molecular weight of 6,000 (0.1% by mass) instead of polyethylene glycol having the molecular weight of 2,000.

### (1-5-3) Example 3

The antibody immobilized membrane was manufactured in the same way as (1-5-1) except using polyethylene glycol having the molecular weight of 8,000 (0.1% by mass) instead of polyethylene glycol having the molecular weight of 2,000.

### (1-5-4) Comparative Example 1

The antibody immobilized membrane was manufactured in the same way as (1-5-1) except applying anti-influenza A type monoclonal antibody for immobilization without mixing with polyethylene glycol having the molecular weight of 2,000.

### (1-5-5) Comparative Example 2

The antibody immobilized membrane was manufactured in the same way as (1-5-1) except using polyethylene glycol having the molecular weight of 1,000 (0.1% by mass) instead of polyethylene glycol having the molecular weight of 2,000.

### (1-5-6) Comparative Example 3

The antibody immobilized membrane was manufactured in the same way as (1-5-1) except using polyethylene glycol having the molecular weight of 20,000 (0.1% by mass) instead of polyethylene glycol having the molecular weight of 2,000.

### (1-5-7) Comparative Example 4

The antibody immobilized membrane was manufactured in the same way as (1-5-1) except using polyvinyl alcohol (PVA) having the molecular weight of 72,500 (0.1% by mass) instead of polyethylene glycol having the molecular weight of 2,000.

### (1-5-8) Comparative Example 5

The antibody immobilized membrane was manufactured in the same way as (1-5-1) except using BSA having the molecular weight of 66,000 (0.1% by mass) instead of polyethylene glycol having the molecular weight of 2,000.

### (1-5-9) Comparative Example 6

The antibody immobilized membrane was manufactured in the same way as (1-5-1) except using dextran having the molecular weight of 40,000 (0.1% by mass) instead of polyethylene glycol having the molecular weight of 2,000.

### (1-5-10) Comparative Example 7

The antibody immobilized membrane was manufactured in the same way as (1-5-1) except using dextran sulfate having the molecular weight of 5,000 (0.1% by mass) instead of polyethylene glycol having the molecular weight of 2,000.

In Examples 1 to 3 and Comparative Examples 1 to 7, the application quantity of the antibody is 0.21 µg/mm², and the application quantity of polyethylene glycol, polyvinyl alcohol, BSA, dextran and dextran sulfate is respectively 0.14 µg/mm². In addition, PEG/antibody (mole ratios) in Examples 1 to 3 and Comparative Examples 1 to 7 are shown in Table 1.

### (1-6) Manufacturing of anti-influenza B type antibody immobilized membrane (chromatography carrier)

### (1-6-1) Example 4

By using a nitrocellulose membrane which was cut by 25 mm×200 mm (there is a plastic-backed, HiFlow Plus HF120, manufactured by EMD Millipore Corporation), an antibody was immobilized by a method as described below to manufacture an antibody immobilized membrane. The long side of the membrane was set to the lower side (the near side), and at a position of 10 mm from the lower side, a solution in which polyethylene glycol having the molecular weight of 8,000 was mixed with anti-influenza B type monoclonal antibody for immobilization (Anti-Influenza B M2110171, manufactured by Fitzgerald Industries International, Inc.) solution which was prepared so as to be 1.5 mg/mL so that the content of polyethylene glycol became 0.17% by mass was applied in a line shape having the width of approximately 0.7 mm by using an ink jet type applying device (manufactured by BioDot, Inc.). This line is called a test line. In the same way, at a position of 13 mm from the lower side, an anti-mouse IgG antibody for control (an anti-mouse IgG (H+L), rabbit F(ab')₂, the stock number 566-70261, manufactured by Wako Pure Chemical Industries, Ltd.) solution which was prepared so as to be 0.5 mg/mL was applied in a line shape. This line is called a control line. The applied membrane was dried at 50°C for 30 minutes by a hot air type dryer. 500 mL of a blocking liquid (50 mM boric acid buffer (pH 8.5) containing 0.5% by mass of casein (derived from the milk, the stock number 030-01505, manufactured by Wako Pure Chemical Industries, Ltd.)) was put into the vat, and the dried membrane was immersed therein and was left to stand for 30 minutes as it was. Afterward, the membrane which was taken out from a blocking liquid was immersed in 500 mL of a washing and stabilized liquid (50 mM Tris-HCl (pH 7.5) buffer containing 0.5% by mass of sucrose and 0.05% by mass of sodium cholate) which was put into another vat and was left to stand for 30 minutes as it was. The membrane was taken out from the liquid and dried at room temperature for 12 hours to set to an antibody immobilized membrane.

### (1-6-2) Comparative Example 8

The antibody immobilized membrane was manufactured in the same way as (1-6-1) except applying anti-influenza B type monoclonal antibody for immobilization without mixing with polyethylene glycol having the molecular weight of 8,000.

### (1-6-3) Comparative Example 9

The antibody immobilized membrane was manufactured in the same way as (1-6-1) except using polyethylene glycol having the molecular weight of 1,000 (0.17% by mass) instead of polyethylene glycol having the molecular weight of 8,000.

### (1-6-4) Comparative Example 10

The antibody immobilized membrane was manufactured in the same way as (1-6-1) except using polyethylene glycol having the molecular weight of 20,000 (0.17% by mass) instead of polyethylene glycol having the molecular weight of 8,000.

### (1-6-5) Comparative Example 11

The antibody immobilized membrane was manufactured in the same way as (1-6-1) except using bovine serum albumin having the molecular weight of 66,000 (BSA, 0.17% by mass) instead of polyethylene glycol having the molecular weight of 8,000.

In Example 4 and Comparative Examples 8 to 11, the application quantity of the antibody is 0.21 µg/mm², and the application quantity of polyethylene glycol and BSA is respectively 0.24 µg/mm². In addition, PEG/antibody (mole ratios) in Examples 4 and Comparative Examples 8 to 11 are shown in Table 2.

### (1-7) Manufacturing of insoluble carrier for chromatography

The antibody immobilized membrane manufactured in (1-5) or (1-6) was respectively stuck to a back adhesive sheet (ARcare9020, manufactured by NIPPN Techno Cluster, Inc.). As to the antibody immobilized membrane to which the back adhesive sheet was adhered, the membrane was set to become horizontally long so that the test line side became the lower side (the near side), and the gold colloid antibody retention pads which were manufactured in (1-3) and (1-4) were stuck so as to overlap approximately 2 mm on the lower side of the antibody immobilized membrane. Furthermore, the sample addition pad (glass fiber pad which was cut by 18 mm×200 mm (Glass Fiber Conjugate Pad, manufactured by EMD Millipore Corporation)) was stuck by overlapping so as to overlap approximately 4 mm on the lower side of the gold colloid antibody retention pad. Furthermore, the absorbent pad (Cellulose-glass membrane which was cut by 80 mm×200 mm (CF6, manufactured by Whatman plc.)) was stuck by overlapping so as to overlap approximately 5 mm on the upper side of the antibody immobilized membrane. A sheet for chromatography manufactured in this manner was cut by the width of 7 mm each to manufacture the insoluble carrier for chromatography.

### (1-8) Manufacturing of washing liquid

23.6 mL of 1 mol/L iron nitrate aqueous solution manufactured by dissolving iron(III) nitrate nonahydrate (the stock number 095-00995, manufactured by Wako Pure Chemical Industries, Ltd.) in water and 13.1 g of citric acid (the stock number 038-06925, manufactured by Wako Pure Chemical Industries, Ltd.) were dissolved in 290 g of water. After all was dissolved, 36 mL of nitric acid (10% by weight) was added while stirring with a stirrer, furthermore, 60.8 g of ammonium iron(II) sulfate hexahydrate (the stock number 091-00855, manufactured by Wako Pure Chemical Industries, Ltd.) was added to obtain a washing liquid. Moreover, the washing liquid is a liquid containing the reducing agent of a silver ion and plays a role of the part of the amplification liquid.

### (1-9) Manufacturing of amplification liquid

8 mL of silver nitrate solution (containing 10g of silver nitrate) and 24 mL of 1 mol/L iron nitrate aqueous solution were added to 66 g of water. Furthermore, the solution was mixed with a solution in which 5.9 mL of nitric acid (10% by weight), 0.1 g of dodecylamine (the stock number 123-00246, manufactured by Wako Pure Chemical Industries, Ltd.) and 0.1 g of a surfactant C₁₂H₂₅-C₆H₄-O-(CH₂CH₂O)₅₀H were dissolved in 47.6 g of water in advance to obtain an amplification liquid.

### (1-10) Manufacturing of device for assay

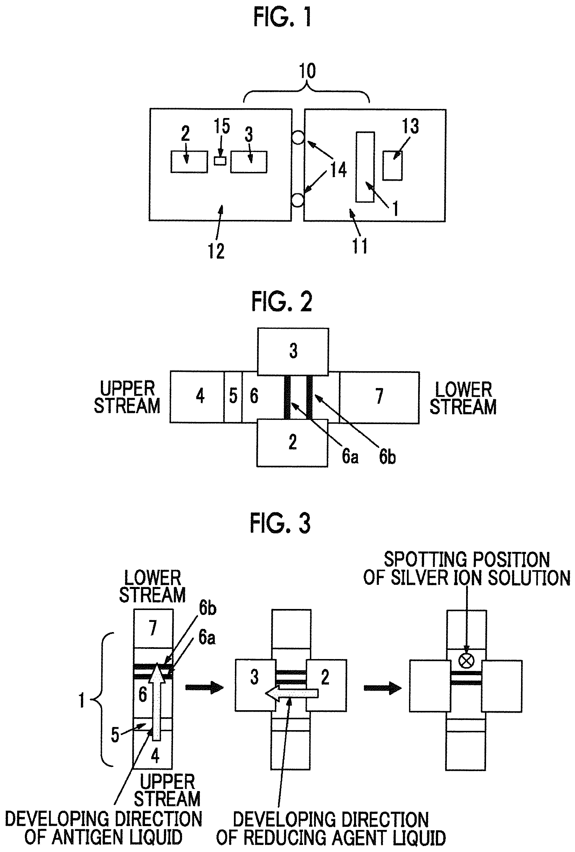

The insoluble carrier for chromatography manufactured in (1-7) was loaded into a first device part (made of polypropylene by injection forming) 11 shown in Fig. 1. Next, a glass fiber pad (Glass Fiber Conjugate Pad, manufactured by EMD Millipore Corporation) was loaded as an insoluble carrier for feeding a liquid 2 and an insoluble carrier for absorbing 3 as shown in Fig. 2.

### (2-1) Evaluation

### (2-1-1) Spotting and developing of test substance (test sample) liquid

A type simulate positive test substance (BD Flu examan control A+B-(manufactured by Becton, Dickinson and Company)) or B type simulate positive test substance (BD Flu examan control A-B+(manufactured by Becton, Dickinson and Company)) was diluted by 1% by mass of BSA-PBS (Phosphate Buffered Saline), and the obtained test substance liquid was spotted with 140 µL from a test substance injected hole to the sample addition pad of the insoluble carrier for chromatography manufactured in (1-7) and after leaving to stand for 10 minutes, the test substance liquid was diffused and developed.

### (2-1-2) Washing

After the test substance liquid was developed for 10 minutes, 200 µL of the washing liquid manufactured in (1-8) was put into a liquid storage pod 13 of the first device part 11 shown in Fig. 1. The first device part 11 was closed by facing to the second device part 12. In this manner, the washing liquid was developed in the insoluble carrier for chromatography to feed a liquid for 2 minutes. According to this step, the insoluble carrier for chromatography was immersed in the washing liquid and a material which was not specifically adsorbed was further washed.

### (2-1-3) Silver amplification

The amplification liquid manufactured in (1-9) from a spotting hole 15 provided in the second device part 12 was added dropwise and the silver amplification reaction was conducted for 1 minute. After amplifying, the insoluble carrier for chromatography was taken out and washed with water for 3 minutes.

### (2-1-4) Calculation of test line concentration value

A picture of the insoluble carrier for chromatography which was washed with water was taken by LAS4000 (manufactured by Fujifilm Corporation), and a test line ΔOD (the optical density (OD) of the test line-the optical density (OD) of the non-test line part) was calculated. ΔOD of the test line in a case where the antigen (the specimen) was included was defined as a signal (S) and ΔOD of the test line in a case where the antigen was not included was defined as a signal (N). In Table 1 and Table 2, in the upper row in the column of each Example and each Comparative Example, the signal (N) which was ΔOD of the test line in a case where the antigen was not included was shown, and in the lower row in the column of each Example and each Comparative Example, the signal (S) which was ΔOD of the test line in a case where the antigen was included was shown. In addition, in Table 1 and Table 2, an S/N ratio as to each Example and each Comparative Example was calculated and described.

(3) As it is clear from the results of Table 1 and Table 2, by mixing PEG having the molecular weight from 1,500 to 10,000 with the antibody to immobilize, it is shown that it is possible to specifically suppress a false positive. In addition, a suppressing effect of a false positive of a case of using PEG having the molecular weight from 1,500 to 10,000 was higher than that of a case of using BSA, PVA or polysaccharide. Furthermore, in a case where there was the antigen, the concentration (sensitivity) of the test line was highly maintained.

**[Table 1]**

| Test Example | Antibody | Mixture | Molecular weight | Antigen | Test line ΔOD (mAbs) | S/N | PEG/Antibody (mole ratio) |
|---|---|---|---|---|---|---|---|
| Comparative Example 1 | A type antibody | None | - | None | 17 | 5.8 | - |
| | | | | A+B-BD Flu examan control 1/2560 diluted | 99 | | |
| Comparative Example 2 | A type antibody | PEG | 1000 | None | 17 | 5.0 | 100.0 |
| | | | | A+B- BD Flu examan control 1/2560 diluted | 85 | | |
| Example 1* | A type antibody | PEG | 2000 | None | 4 | 21.6 | 50.0 |
| | | | | A+B-BD Flu examan control 1/2560 diluted | 90 | | |
| Example 2* | A type antibody | PEG | 6000 | None | 4 | 24.3 | 16.7 |
| | | | | A+B-BD Flu examan control 1/2560 diluted | 100 | | |
| Example 3 | A type antibody | PEG | 8000 | None | 1 | 71.3 | 12.5 |
| | | | | A+B-BD Flu examan control 1/2560 diluted | 63 | | |
| Comparative Example 3 | A type antibody | PEG | 20000 | None | 5 | 10.8 | 5.0 |
| | | | | A+B- BD Flu examan control 1/2560 diluted | 56 | | |
| Comparative Example 4 | A type antibody | PVA | 72500 | None | 12 | 6.9 | - |
| | | | | A+B- BD Flu examan control 1/2560 diluted | 86 | | |
| Comparative Example 5 | A type antibody | BSA | 66000 | None | 11 | 8.2 | - |
| | | | | A+B- BD Flu examan control 1/2560 diluted | 88 | | |
| Comparative Example 6 | A type antibody | Dextran (polysaccharide) | 40000 | None | 17 | 5.7 | - |
| | | | | A+B- BD Flu examan control 1/2560 diluted | 99 | | |
| Comparative Example 7 | A type antibody | Dextran sulfate (polysaccharide) | 5000 | None | 18 | 5.6 | - |
| | | | | A+B- BD Flu examan control 1/2560 diluted | 100 | | |

| | | | | | | | |
|---|---|---|---|---|---|---|---|
| *Reference | | | | | | | |

**[Table 2]**

| Test Example | Antibody | Mixture | Molecular weight | Antigen | Test line ΔOD (mAbs) | S/N | PEG/Antibody (mole ratio) |
|---|---|---|---|---|---|---|---|
| Comparative Example 8 | B type antibody | None | - | None | 66 | 1.4 | - |
| | | | | A-B+BD Flu examan control 1/4100 diluted | 93 | | |
| Comparative Example 9 | B type antibody | PEG | 1000 | None | 51 | 1.8 | 170.0 |
| | | | | A-B+BD Flu examan control 1/4100 diluted | 93 | | |
| Example 4 | B type antibody | PEG | 8000 | None | 11 | 9.1 | 21.3 |
| | | | | A-B+BD Flu examan control 1/4100 diluted | 101 | | |
| Comparative Example 10 | B type antibody | PEG | 20000 | None | 6 | 2.4 | 8.5 |
| | | | | A-B+BD Flu examan control 1/4100 diluted | 14 | | |
| Comparative Example 11 | B type antibody | BSA | 66000 | None | 35 | 2.0 | - |
| | | | | A-B+BD Flu examan control 1/4100 diluted | 71 | | |

## Claims

1. A chromatography method comprising:
developing a complex of a specimen and a labeled substance modified by a first bondable substance which can be bonded to the specimen, on an insoluble carrier;
capturing the complex of the specimen and the labeled substance at a reactive site on the insoluble carrier;
washing the reactive site on the insoluble carrier using a washing liquid; and
detecting the specimen which is captured at the reactive site after a signal of the labeled substance is amplified by an amplification liquid,
wherein the reactive site on the insoluble carrier includes
(i) a second bondable substance which can be bonded to the specimen or a substance having affinity to the first bondable substance which can be bonded to the specimen, and
(ii) polyethylene glycol having the molecular weight from 7,000 to 9,000,
wherein each of the first bondable substance and the second bondable substance is an antibody, and
wherein the mole ratio of the polyethylene glycol and the second bondable substance which can be bonded to the specimen or the substance having affinity to the first bondable substance which can be bonded to the specimen is from 12.5 to 21.3.

2. The chromatography method according to claim 1,
wherein the reactive site on the insoluble carrier is provided by applying a mixed liquid including
(i) the second bondable substance which can be bonded to the specimen or the substance having affinity to the first bondable substance which can be bonded to the specimen; and
(ii) the polyethylene glycol
onto the insoluble carrier.

3. The chromatography method according to claim 1 or 2,
wherein the amplification liquid includes a compound including silver.

4. The chromatography method according to any one of claims 1 to 3,
wherein the washing liquid includes a reducing agent which can reduce a silver ion.

5. The chromatography method according to any one of claims 1 to 4,
wherein the reducing agent which can reduce a silver ion includes a divalent iron ion.

6. The chromatography method according to any one of claims 1 to 5,
wherein the labeled substance is a metal colloid.

7. A chromatography kit comprising:
(a) a labeled substance modified by a first bondable substance which can be bonded to a specimen;
(b) an insoluble carrier provided with a reactive site including (i) a second bondable substance which can be bonded to the specimen or a substance having affinity to the first bondable substance which can be bonded to the specimen, and (ii) polyethylene glycol having the molecular weight from 7,000 to 9,000;
wherein each of the first bondable substance and the second bondable substance is an antibody, and
wherein the mole ratio of the polyethylene glycol and the second bondable substance which can be bonded to the specimen or the substance having affinity to the first bondable substance which can be bonded to the specimen is from
12.5 to 21.3;
(c) a washing liquid; and
(d) an amplification liquid.

8. A method of producing an insoluble carrier for chromatography comprising:
applying a mixed liquid including
(i) a second bondable substance which can be bonded to a specimen or a substance having affinity to a first bondable substance which can be bonded to the specimen and
(ii) polyethylene glycol having the molecular weight from 7,000 to 9,000 onto an insoluble carrier,
wherein each of the first bondable substance and the second bondable substance is an antibody, and
wherein the mole ratio of the polyethylene glycol and the second bondable substance which can be bonded to the specimen or the substance having affinity to the first bondable substance which can be bonded to the specimen is from
12.5 to 21.3.

## Patentansprüche

1. Chromatographie-Verfahren, enthaltend:
Entwickeln eines Komplexes aus einer Probe und einer markierten Substanz, die durch eine erste bindefähige Substanz modifiziert ist, die an die Probe gebunden werden kann, auf einem unlöslichen Träger,
Einfangen des Komplexes aus der Probe und der markierten Substanz an einer reaktiven Stelle auf dem unlöslichen Träger,
Waschen der reaktiven Stelle auf dem unlöslichen Träger unter Verwendung einer Waschlösung; und
Ermitteln der Probe, die an der reaktiven Stelle eingefangen ist, nachdem ein Signal der markierten Substanz durch eine Amplifizierflüssigkeit amplifiziert ist,
worin die reaktive Stelle auf dem unlöslichen Träger enthält
(i) eine zweite bindefähige Substanz, die an die Probe gebunden werden kann, oder eine Substanz mit einer Affinität zu der ersten bindefähigen Substanz, die an die Probe gebunden werden kann, und
(ii) Polyethylenglycol mit einem Molekulargewicht von 7000 bis 9000,
worin jede der ersten bindefähigen Substanz und der zweiten bindefähigen Substanz jeweils ein Antikörper ist und
worin das Molverhältnis des Polyethylenglycols und der zweiten bindefähigen Substanz, die an die Probe gebunden werden kann, oder der Substanz mit einer Affinität für die erste bindefähige Substanz, die an die Probe gebunden werden kann, von 12,5 bis 21,3 ist.

2. Chromatographie-Verfahren nach Anspruch 1, worin die reaktive Stelle auf dem unlöslichen Träger vorgesehen wird durch Auftragen einer gemischten Flüssigkeit, die enthält:
(i) die zweite bindefähige Substanz, die an die Probe gebunden werden kann, oder die Substanz mit einer Affinität für die erste bindefähige Substanz, die an die Probe gebunden werden kann, und
(ii) das Polyethylenglycol,
auf den unlöslichen Träger.

3. Chromatographie-Verfahren nach Anspruch 1 oder 2, worin die Amplifizierflüssigkeit eine Verbindung enthält, die Silber enthält.

4. Chromatographie-Verfahren nach einem der Ansprüche 1 bis 3, worin die Waschflüssigkeit ein Reduktionsmittel enthält, das ein Silberion reduzieren kann.

5. Chromatographie-Verfahren nach einem der Ansprüche 1 bis 4, worin das Reduktionsmittel, das ein Silberion reduzieren kann, ein bivalentes Eisenion enthält.

6. Chromatographie-Verfahren nach einem der Ansprüche 1 bis 5, worin die markierte Substanz ein Metallkolloid ist.

7. Chromatographie-Kit, enthaltend:
(a) eine markierte Substanz, modifiziert durch eine erste bindefähige Substanz, die an eine Probe gebunden werden kann,
(b) einen unlöslichen Träger, der mit einer reaktiven Stelle versehen ist, enthaltend (i) eine zweite bindefähige Substanz, die an die Probe gebunden werden kann, oder eine Substanz mit einer Affinität für die erste bindefähige Substanz, die an die Probe gebunden werden kann, und (ii) Polyethylenglycol mit einem Molekulargewicht von 7000 bis 9000,
worin jede der ersten bindefähigen Substanz und der zweiten bindefähigen Substanz ein Antikörper ist und
worin das Molverhältnis von Polyethylenglycol und der zweiten bindefähigen Substanz, die an die Probe gebunden werden kann, oder der Substanz mit einer Affinität für die erste bindefähige Substanz, die an die Probe gebunden werden kann, von 12,5 bis 21,3 ist,
(c) eine Waschlösung und
(d) eine Amplifizierflüssigkeit.

8. Verfahren zur Erzeugung eines unlöslichen Trägers für die Chromatographie, enthaltend:
Auftragen einer gemischten Lösung, enthaltend
(i) eine zweite bindefähige Substanz, die an eine Probe gebunden werden kann, oder eine Substanz mit einer Affinität zu einer ersten bindefähigen Substanz, die an die Probe gebunden werden kann, und
(ii) Polyethylenglycol mit einem Molekulargewicht von 7000 bis 9000,
auf den unlöslichen Träger,
worin jede der ersten bindefähigen Substanz und der zweiten bindefähigen Substanz ein Antikörper ist und worin das Molverhältnis von Polyethylenglycol und der zweiten bindefähigen Substanz, die an die Probe gebunden werden kann, oder der Substanz mit einer Affinität für die erste bindefähige Substanz, die an die Probe gebunden werden kann, von 12,5 bis 21,3 ist.

## Revendications

1. Procédé de chromatographie comprenant :
le développement d'un complexe d'un spécimen et d'une substance marquée modifié par une première substance liable qui peut être liée au spécimen, sur un support insoluble ;
la capture du complexe du spécimen et de la substance marquée au niveau d'un site réactif sur le support insoluble ;
le lavage du site réactif sur le support insoluble en utilisant un liquide de lavage ; et
la détection du spécimen qui est capturé au niveau du site réactif après qu'un signal de la substance marquée est amplifié par un liquide d'amplification,
dans lequel le site réactif sur le support insoluble inclut
(i) une seconde substance liable qui peut être liée au spécimen ou une substance ayant une affinité pour la première substance liable qui peut être liée au spécimen, et
(ii) un polyéthylène glycol ayant le poids moléculaire de 7000 à 9000,
dans lequel chacune parmi la première substance liable et la seconde substance liable est un anticorps, et
dans lequel le rapport molaire entre le polyéthylène glycol et la seconde substance liable qui peut être liée au spécimen ou la substance ayant une affinité pour la première substance liable qui peut être liée au spécimen est de 12,5 à 21,3.

2. Procédé de chromatographie selon la revendication 1,
dans lequel le site réactif sur le support insoluble est fourni par l'application d'un liquide mixte incluant
(i) la seconde substance liable qui peut être liée au spécimen ou la substance ayant une affinité pour la première substance liable qui peut être liée au spécimen ; et
(ii) le polyéthylène glycol
sur le support insoluble.

3. Procédé de chromatographie selon la revendication 1 ou 2,
dans lequel le liquide d'amplification inclut un composé incluant de l'argent.

4. Procédé de chromatographie selon l'une quelconque des revendications 1 à 3,
dans lequel le liquide de lavage inclut un agent réducteur qui peut réduire un ion d'argent.

5. Procédé de chromatographie selon l'une quelconque des revendications 1 à 4,
dans lequel l'agent réducteur qui peut réduire un ion d'argent inclut un ion de fer divalent.

6. Procédé de chromatographie selon l'une quelconque des revendications 1 à 5, dans lequel la substance marquée est un colloïde métallique.

7. Kit de chromatographie comprenant :
(a) une substance marquée modifiée par une première substance liable qui peut être liée à un spécimen ;
(b) un support insoluble pourvu d'un site réactif incluant (i) une seconde substance liable qui peut être liée au spécimen ou une substance ayant une affinité pour la première substance liable qui peut être liée au spécimen, et (ii) un polyéthylène glycol ayant le poids moléculaire de 7000 à 9000 ;
dans lequel chacune parmi la première substance liable et la seconde substance liable est un anticorps, et
dans lequel le rapport molaire entre le polyéthylène glycol et la seconde substance liable qui peut être liée au spécimen ou la substance ayant une affinité pour la première substance liable qui peut être liée au spécimen est de 12,5 à 21,3 ;
(c) un liquide de lavage ; et
(d) un liquide d'amplification.

8. Procédé de production d'un support insoluble pour chromatographie comprenant :
l'application d'un liquide mixte incluant
(i) une seconde substance liable qui peut être liée à un spécimen ou une substance ayant une affinité pour une première substance liable qui peut être liée au spécimen et
(ii) un polyéthylène glycol ayant le poids moléculaire de 7000 à 9000 sur un support insoluble,
dans lequel chacune parmi la première substance liable et la seconde substance liable est un anticorps, et
dans lequel le rapport molaire entre le polyéthylène glycol et la seconde substance liable qui peut être liée au spécimen ou la substance ayant une affinité pour la première substance liable qui peut être liée au spécimen est de 12,5 à 21,3.
